(19) Europäisches Patentamt
European Patent Office
Office européen des brevets

(11) Publication number: 0 195 575 B1

(12) **EUROPEAN PATENT SPECIFICATION**

(45) Date of publication of the patent specification: 27.12.89

(51) Int. Cl.⁴: A61K 7/027

(21) Application number: 86301694.5

(22) Date of filing: 10.03.86

(54) **Improved lipstick formulation and method.**

(30) Priority: 21.03.85 US 714646

(43) Date of publication of application: 24.09.86 Bulletin 86/39

(45) Publication of the grant of the patent: 27.12.89 Bulletin 89/52

(84) Designated Contracting States: AT BE CH DE FR GB IT LI LU NL SE

(56) References cited:
AT-A- 323 328
DE-A- 2 427 487
DE-A- 3 427 254
DE-B- 2 646 675
GB-A- 2 008 943
US-A- 3 574 822
US-A- 3 927 203

(73) Proprietor: CHARLES OF THE RITZ GROUP LTD., 40 West 57th Street, New York, NY 10019(US)

(72) Inventor: Mercado, Clara Garcia, 212 Wellington Drive, Aberdeen New Jersey(US)
Inventor: Krog, Ann Marshall, 36 Rutledge Drive, Red Bank New Jersey(US)

(74) Representative: Thomas, Roger Tamlyn et al, D. Young & Co. 10 Staple Inn, London WC1V 7RD(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 195 575 B1

ACTORUM AG

## Description

The present invention relates to a new lipstick formulation which has unique color laydown properties primarily due to the presence therein of an acrylates copolymer powder.

Lipsticks have been used for many years to impart color to the lips. The color helps to define the mouth area while imparting cosmetic shades that are suitable with fashion trends. Such lipsticks, in general, are made of an oily vehicle comprising fat or oil stiffened to a desired consistency with waxes of various types which also serve to raise the melting point and improve the physical stability. The color is ordinarily provided by insoluble pigments such as lakes of dye finely dispersed in the oily vehicle and one or more fluorescein dye derivatives which serve to stain the lips. A solvent for the dye is also included for increasing the effectiveness of this staining on the lips.

In recent years, attemps have been made to provide a lipstick which will impart a uniform long-lasting coloring to the lips and thus lessen the need for frequent reapplication. This has been accomplished by increasing the concentration of the dye and pigment in the conventional lipstick formulation to greater than 10% by weight. However, lipsticks containing such high concentrations of dye and pigment have been found to impart a dry non-uniform "cakey" look to the lips. This is particularly undesirable since current fashion trends are directed to producing a "we" or "moist" look on the mouth. As a result, emollients, lubricants, and moisturizers are added in increased amount to counteract the "cakey" results of the high dye and pigment containing lipstick formulations. However, these materials may impart a soft mushy easily breakable structure to the lipstick. Furthermore, these materials themselves are usually short-lived on the mouth since they are mechanically removed. In addition, these materials tend to act as vehicles for the dyes and pigments causing "creeping" or "feathering" on the outer edges of the mouth. This eliminates the sharp line of definition of the mouth area for which the product is applied.

US Patent US–A 3,911,105 discloses lipstick compositions which contain polymers having the repeating unit $-CH_2-C(R_1)(COOR_2)-$ where $R_1$ is H or $CH_3$ and $R_2$ is a saturated linear or branched hydrocarbon group of 10–20 carbon atoms. The lipsticks containing these polymers are said to have improved resistance to breaking, brilliance and holding power of the film deposited on the lips.

As hereinafter described, a new creamy soft lipstick formulation is provided which has excellent laydown properties so that it may impart uniform long-lasting coloring to the lips and has excellent structural integrity properties so it will not easily break or become mushy, but stays intact. Furthermore, the lipstick formulation does not contain inordinately high amounts of pigment and/or dye so that it will not impart an undesirable dry "cakey" look to the lips. The improved laydown properties of the lipstick formulation is achieved, at least in part, through the inclusion of an acrylate polymer powder therein.

According to one aspect, the invention provides a lipstick formulation which is soft but has good stick structure, is creamy and shiny, and has good laydown and color-wear properties, said formulation comprising from about 40 to about 80% by weight anhydrous base intermediate comprising one or more waxes and one or more oils; from about 8 to about 35% by weight of a color intermediate which includes an oil dispersing agent; and an anhydrous acrylate polymer powder dispersion intermediate comprising an acrylate polymer powder and oil dispersing agent for said acrylate polymer powder, wherein the acrylate polymer (i) has the repeating unit

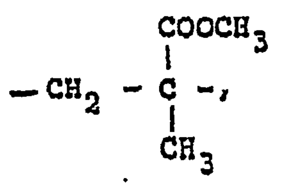
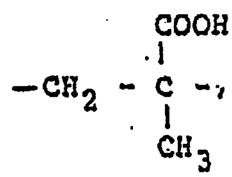

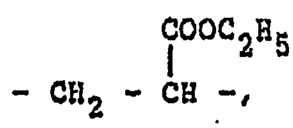
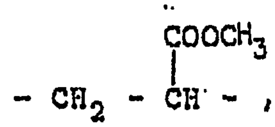

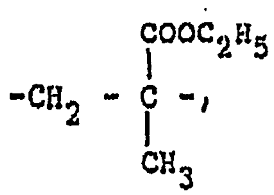
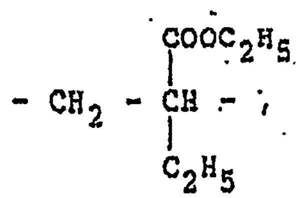

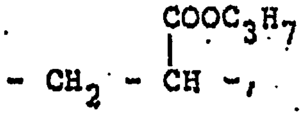

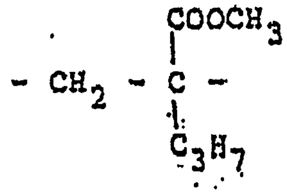

, or

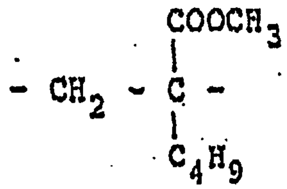

(ii) has an average molecular weight within the range of from about 100,000 to about 500,000, and (iii) is present in an amount within the range of from about 0.1 to about 4% by weight of the lipstick formulation.

According to another aspect, the invention provides a method for preparing an improved lipstick formulation which has good laydown properties, creaminess and shininess, and good color wear and stick strength, said method comprising incorporating in an anhydrous lipstick formulation (A) an anhydrous acrylate polymer powder dispersion intermediate comprising (a) an acrylate polymer powder wherein the acrylate polymer (i) has the repeating unit

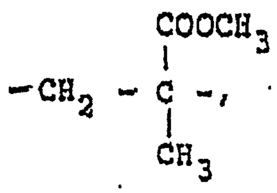

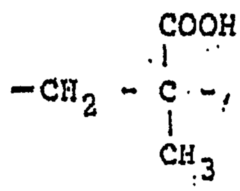

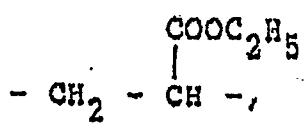

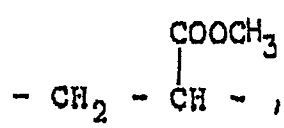

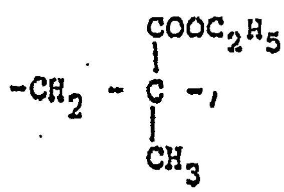

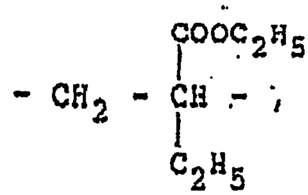

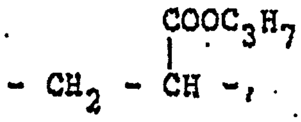

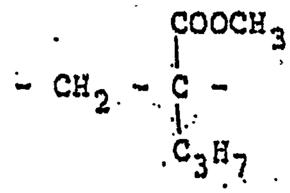

, or

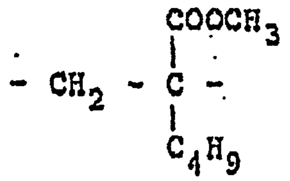

(ii) has an average molecular weight within the range of from about 100,000 to about 500,000, and (iii) is present in an amount within the range of from about 0.1 to about 4% by weight of the lipstick formulation, and (b) an oil dispersing agent for said acrylate polymer powder, and (B) from about 8 to about 35% by weight of a color intermediate which includes an oil dispersing agent.

The preferred lipstick formulation of the invention is formed of from about 40 to about 80% and preferably from about 50 to about 70% by weight of anhydrous base intermediate which will be fully described hereinafter; from about 3 to about 20% by weight and preferably from about 5 to about 15% by weight of one or more dispersing oils for organic pigments, from about 8 to about 35% by weight and preferably from about 12 to about 30% by weight of color intermediate comprised of a mixture of pigments, dyes or colors and one or more dispersing oils or other softeners, optionally antioxidant and optionally perservative; from about 0.5 to about 5% by weight and preferably from about 1 to about 3% by weight of the acrylate polymer intermediate, which includes from about 0.1 to about 4% and preferably from about 0.2 to about 2.5% by weight of the acrylate polymer by itself to impart and enhance laydown properties of the formulation, and optionally from about 0 to about 1% and preferably from about 0.1 to about 0.8% by weight fragrance, all of the above % being based on the total lipstick formulation. The ingredients of the preferred formulation will now be described in more detail.

The acrylate polymer intermediate will generally be formed of from about 20 to about 80% by weight acrylate polymer powder and from about 80 to about 20% by weight of one or more dispersants therefor, such as castor oil, mineral oil, isopropyl isostearate, oleyl alcohol, liquid lanolin, sesame oil, isopropyl myristate, isopropyl palmitate, squalene and the like, with castor oil being preferred.

The acrylate polymer powder will preferably have an average particle size of within the range of from about 270 to about 325 mesh (0.044–0.053 mm sieve opening).

The preferred acrylate polymers have the repeating unit

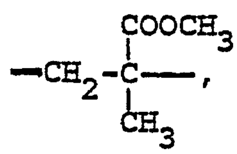

,

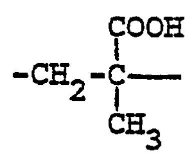

or

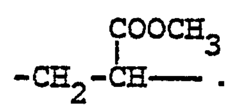

.

A most preferred acrylate polymer powder is Polytrap 249 polymer powder which has the repeating unit formula

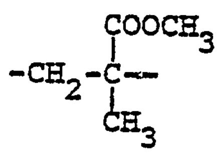

which has an average molecular weight within the range of from about 100,000 to about 500,000 and is marketed by Wickhen Products, Incorporated, Chemical Specialty Products, Huguenot, New York and has a bulk density of from about 0.52 to about 0.57, a flash point of greater than 300°F (149°C) and a particle size so that about 30% is retained on a 270 mesh screen and about 4% is retained on a 325 mesh screen.

The anhydrous base intermediate will generally be formed of one or more external stick strengtheners in an amount of within the range of from about 5 to about 20% by weight and preferably from about 8 to

about 16% by weight based on the weight of the total lipstick formulation. Such external stick strengtheners provide one or more of the follwing properties: impart structural intergrity to the lipstick, provide hardness, provide mold release properties, and stick strength and drag on the lips. Examples of external stick strengtheners suitable for use· herein include waxes such as candelilla, ozokerite, carnauba, beeswax and the like or mixture of two or more of such waxes.

One or more wax feel enhancers and structure strentheners will also be included in the base intermediate in an amount within the range of from about 5 to about 20% by weight and preferably from about 9 to about 16% by weight of the total lipstick formulation. Examples of such wax feel enhancers and structure strengtheners include cetyl alcohol, stearyl alcohol, lanolin USP, super wool wax (a lanolin alcohol fraction), glyceryl monostearate and the like or mixtures thereof.

The best intermediate will also contain an internal stick strengthener in an amount within the range of from about 0.5 to about 10% by weight and preferably from about 1 to about 6% by weight based on the total stick formulation. A preferred internal stick strengthener is paraffin wax. Other examples are ozokerite wax or carnauba wax.

One or more feel enhancers in cream form will also be included in the base intermediate in an amount within the range of from about 1 to about 10% by weight and preferably from about 2 to about 7% by weight based on the total stick formulation. Examples of suitable cream feel enhancers include hydrogenated vegetable oil, isopropyl lanolate, synthetic cocoa butter, acetylated lanolin alcohol and lanolin derivatives or mixtures thereof.

One or more lip moisturizers, emollients, lubricants and/or glide or slip agents will be present, such as petrolatum white (which is a slip moisturizer), oleyl alcohol (a penetrant), castor oil, low viscosity mineral oils (which are glide or slip agents, emollients and/or moisturuzers), liquid lanolin, and/or jojoba oil (which are shine enhancers), isopropyl myristate, isopropyl palmitate, squaline and/or sesame oil (which are lubricants), and the like will also be present in the base intermediate in amounts within the range of from about 3 to about 30% by weight and preferably from about 5 to about 25% by weight based on the total weight of the stick formulation. It is preferred to employ a mixture of these oils for their different functions.

A coupling agent for waxes and oils will also be present in the anhydrous base intermediate in an amount within the range of from about 6 to about 20% by weight and preferably from about 8 to about 15% by weight of the total formulation. Examples of suitable coupling agents include oleyl alcohol, dipropyl diperlargonate or caster oil or mixtures thereof.

The base intermediate may optionally include one or more preservatives in amounts of less than about 0.3% and preferably less than about 0.2% by weight of the total lipstick formulation, such as propyl p-hydroxybenzoate (propyl paraben), methyl p-hydroxybenzoate (methyl paraben) and/or butyl p-hydroxybenzoate (butyl paraben), and optionally less than about 0.05% by weight of an antioxidant such as butylated hydroxyanisole, or Vitamin E tocopherol, and other moisturizers such as wheat germ glycerides and hydrolyzed animal protein.

The color intermediate will contain from about 25 to about 40% by weight of one or more dyes and/or pigments, from about 55 to about 75% by weight and preferably from about 60 to about 70% by weight of one or more color dispersants, such as any of those set out above and used for dispersing the acrylate polymer, with castor oil being preferred, optionally less than about 0.05% and preferably less than about 0.03% by weight antioxidant, such as set out above with respect to the base intermediate, and optionally less than about 0.3% by weight and preferably less than about 0.2% by weight preservative, such as set out above with respect to the base intermediate, all of the above percentages being base on the total weight of the color intermediate.

The dyes employed in the formulation are the U.S. Government certified colors, both Drug and Cosmetic grade, and Food, Drug and Cosmetic grade. The pigments employed are of the inorganic grade such as iron oxides, titanium dioxide, iron sulfides, or other conventional pigments approved for cosmetic use.

Anhydrous natural flavor and fragrance oils such as peppermint oil, lemon oil, orange oil, etc. or synthetic flavor and fragrance oils may optionally be included in the lipstick formulation by incorporation into the anhydrous base or even into the color intermediate, in amounts of less than about 1% by weight of the lipstick formulation and preferably from about 0.1 to about 0.8% by weight.

The lipstick when applied coats the lips with the acrylate polymer causing the color or pigment to easily laydown in a creamy uniform manner with the colors penetrating every pore and crevice on the lip surface.

Preferred lipstick formulations in accordance with the present invention are set out below.

Preferred Formulations

| Ingredient | % w/w of Total Lipstick Formulation |
|---|---|
| Base Intermediate | 50 to 70 |
| Candelilla wax (stick strengthener) | 10 to 14 |
| Cetyl alcohol (feel enhancer) | 2 to 6 |
| Ozokerite (paraffin wax – hardness internal stick strengthener) | 1.5 to 10 |
| Lanolin alcohol (feel enhancer) | 7 to 20 |
| Beeswax (strengthener) | 0.5 to 5 |
| Hydrogenated vegetable oil (feel enhancer-cream) | 3 to 6 |
| Petrolatum white (lip moistener) | 1.5 to 12 |
| Lanolin oil (shine enhancer) | 4. to 8 |
| Mineral oil (glide or slip agent) | 4 to 8 |
| Oleyl alcohol (coupling agent for wax and oils) | 7 to 15 |
| Preservative | 0 to 0.1 |
| Antioxidant | 0 to 0.02 |
| Color Intermediate | 10 to 30 |
| Pigments and castor oil dispersant (1:4 to 1:1) | 20 to 34 |
| Antioxidant | 0 to 0.01 |
| Preservative | 0 to 0.05 |
| Softener (castor oil) | 6 to 20 |
| Acrylate Polymer Phase | 1 to 3 |
| Acrylate polymer | |
| Dispersant (castor oil) (1:4 to 4:1) | |
| Fragrance | 0.1 to 0.8 |

The lipstick formulation of the invention may be prepared by forming a mixture of the anhydrous base intermediate ingredients wherein the waxes are melted and the oil components are added with agitation to form the anhydrous base intermediate. The base intermediate and softener (castor oil) are heated to 75 to 85°C with propeller mixing until a liquid is formed.

The color intermediate phase (formed by adding the color to the dispersant, heating with agitation at 65 to 75°C and then roller milling three times) is added to the base intermediatesoftener phase with mixing for 40 to 80 minutes.

Next, the acrylate polymer intermediate (formed by adding the polymer to the dispersant and roller milling 3 times) is added to the mix and mixing is continued for 20 to 35 minutes until a uniform mixture is obtained. Fragrance (where present) is then added and mixing is continued for 10 minutes.

The mixture is then molded to form the lipstick formulation of the invention.

The following Examples represent preferred embodiments of the present invention.

Example 1

A lipstick formulation having the following composition is prepared as described below.

| Ingredient | Parts by Weight |
|---|---|
| Base intermediate | 60 |
| Candelilla wax | 12 |
| Cetyl alcohol | 4 |
| Ozokerite | 2.3 |
| Lanolin alcohol | 9.2 |
| Beeswax | 5 |
| Hydrogenated vegetable oil | 2.5 |
| Petrolatum white | 6.4 |
| Lanolin oil | 6.4 |
| Oleyl alcohol | 11.6 |
| Propyl paraben | 0.09 |
| Butylated hydroxyanisole | 0.02 |
| Softener castor oil | 7.46 |
| Color intermediate | |
| Red 7 | 4 |
| Russet | 2.5 |
| Red 6 | 1 |
| $TiO_2$ | 2 |
| Castor oil | 20 |
| Propyl paraben | 0.005 |
| Butylated hydroxyanisole | 0.025 |
| Acrylate Polymer Intermediate | |
| Acrylate polymer (Polytrap 249 polymer powder) | 1.5 |
| Castor oil | 1.5 |
| Fragrance | 0.5 |

1. All of the waxes were melted in a steam-jacketed kettle with propeller mixer agitation while maintaining the temperature at from 75 to 85°C. After the waxes were melted, the oil components except for the castor oil and the remaining components of the base intermediate were added and agitation was continued.
2. The dyes, pigments, preservative and antioxidant were added to the castor oil in a separate kettle and heated with agitation at 70°C. Agitation was continued until a proper dispersion achieved. The dispersion of oil and coloring agent were passed through a three-roll mill. The color dispersion was added to the base intermediate with mixing for 40 to 60 minutes.
3. The acrylate polymer dispersed in castor oil and roller milled three times was added to the above mixture with mixing for 20 to 30 minutes until a uniform mixture was obtained.
4. Fragrance was then added with mixing for 10 minutes.

The resulting lipstick formulation was poured into molds and refrigerated to form the lipstick.

It was found that the lipstick formulation of the invention incorporating the acrylate polymer powder was soft but had good structure, was creamy, shiny and had excellent laydown.

Example 2

Following the procedure of Example 1, a lipstick formulation was prepared having the following composition.

| Ingredient | Parts by Weight |
|---|---|
| Base intermediate (48 parts) | |
| Candelilla wax | 9.2 |
| Ozokerite | 4.5 |
| Lanolin alcohol | 9.2 |
| Acetylated lanolin alcohol | 7 |
| Hydrolyzed animal protein | 0.25 |
| Petrolatum white | 9.2 |
| Wheat germ glycerides | 0.45 |
| Vitamin E (dl-alpha tocopherol) | 0.03 |
| Oleyl alcohol | 8 |
| Propyl paraben | 0.2 |
| Softener-castor oil | 19.14 |
| Color intermediate | |
| Red 7 | 4 |
| Russet | 2.5 |
| Red 6 | 1 |
| $TiO_2$ | 2 |
| Castor oil | 20 |
| Propyl paraben | 0.005 |
| Butylated hydroxyanisole | 0.025 |
| Acrylate Polymer Intermediate | |
| Acrylate polymer (Polytrap 249 polymer powder) | 1.5 |
| Castor oil | 1.5 |
| Fragrance | 0.3 |

It was found that the lipstick formulation of the invention incorporating the acrylate polymer powder was soft but had good structure, was creamy, shiny and had excellent color laydown.

## Claims

1. A lipstick formulation which is soft but has good stick structure, is creamy and shiny, and has good laydown and color-wear properties, said formulation comprising from about 40 to about 80% by weight anhydrous base intermediate comprising one or more waxes and one or more oils; from about 8 to about 35% by weight of a color intermediate which includes an oil dispersing agent; and an anhydrous acrylate polymer powder dispersion intermediate comprising an acrylate polymer powder and an oil dispersing agent for said acrylate polymer powder, wherein the acrylate polymer (i) has the repeating unit

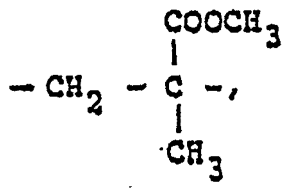

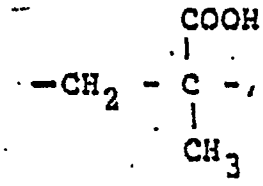

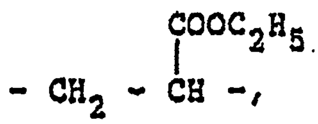

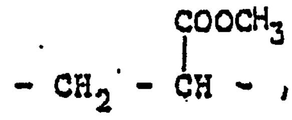

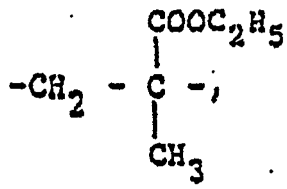

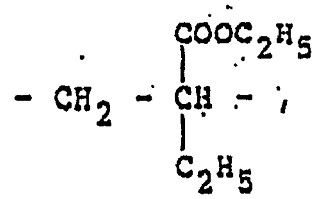

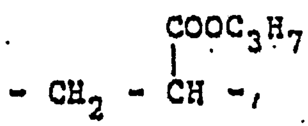

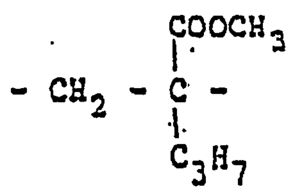

, or

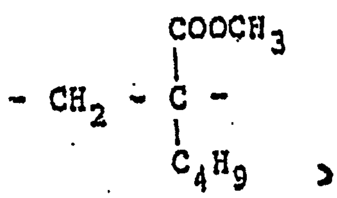

(ii) has an average molecular weight within the range of from about 100,000 to about 500,000, and (iii) is present in an amount within the range of from about 0.1 to about 4% by weight of the lipstick formulation.

2. The lipstick formulation as defined in claim 1 wherein the acrylate polymer is present in an amount within the rage of from about 0.2 to about 2.5% by weight of the lipstick formulation.

3. The lipstick formulation as defined in claim 1 or 2 wherein the acrylate polymer has the repeating unit

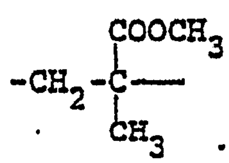

4. The lipstick formulation as defined in any one of claims 1–3 wherein the anhydrous base intermediate is present in an amount within the range of from about 50 to about 70% by weight of the lipstick formulation.

5. The lipstick formulation as defined in any one of Claims 1–4 wherein the base intermediate includes one or more external stick strengtheners, one or more wax feel enhancers and structure strengtheners, one or more internal stick stengtheners, one or more feel enhancers in cream form, one or more lip moisturizers, emollients, lubricants and/or glide or slip agents, one or more coupling agents for waxes and oils, optionally one or more preservatives and optionally one or more antioxidants.

6. The lipstick formulation as defined in Claim 5 wherein the external stick strengthener is present in

an amount within the range of from about 5 to about 20% by weight of the total formulation and is candelilla wax, paraffin wax, carnauba wax, beeswax or a mixture thereof.

7. The lipstick formulation as defined in Claim 5 or 6 wherein the wax feel enhancer and structure strengthener is present in an amount within the range of from about 5 to about 18% by weight of the total formulation and is cetyl alcohol, stearyl alcohol, lanolin, lanolin alcohol, glyceryl monostearate or a mixture thereof.

8. The lipstick formulation as defined in Claim 5, 6 or 7 wherein the internal stick strengthener is present in an amount within the range of from about 0.5 to about 5% by weight of the total formulation and is paraffin wax, carnauba wax or ozokerite wax.

9. The lipstick formulation as defined in Claim 5, 6, 7 or 8 wherein the feel enhancer in cream form is present in an amount within the range of from about 1 to about 10% by weight of the total formulation and is hydrogenated vegetable oil, isopropyl lanolate, cocoa butter, acetylated lanolin alcohol, lanolin derivatives or a mixture thereof.

10. The lipstick formulation as defined in Claim 5, 6, 7, 8 or 9 wherein the lip moisturizers, emollients, lubricants and/or glide or slip agents are present in an amount within the range of from about 3 to about 30% based on the total formulation and are petrolatum white, oleyl alcohol, castor oil, low viscosity mineral oil, liquid lanolin, jojoba oil, isopropyl myristate, isopropyl palmitate, squalane, sesame oil or a mixture thereof.

11. The lipstick formulation as defined in Claim 5, 6, 7, 8, 9 or 10 wherein the coupling agent for waxes and oils is present in an amount within the range of from about 6 to about 15% by weight of the total formulation and is oleyl alcohol, dipropyl dipelargonate, castor oil or a mixture thereof.

12. The lipstick formulation as defined in any preceding claim wherein said oil dispersing agent is castor oil, mineral oil, isopropyl isostearate, oleyl alcohol, liquid lanolin, sesame oil, isopropyl myristate, isopropyl palmitate, or squalene.

13. A method for preparing an improved lipstick formulation which has good laydown properties, creaminess and shininess, and good color wear and stick strength, said method comprising incorporating in an anhydrous lipstick formulation (A) an anhydrous acrylate polymer powder dispersion intermediate comprising (a) an acrylate polymer powder wherein the acrylate polymer (i) has the repeating unit

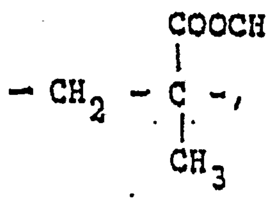

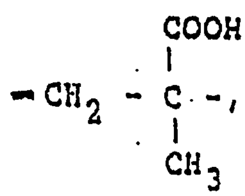

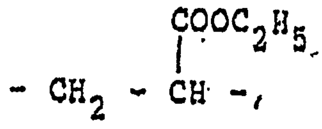

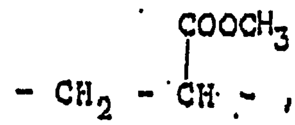

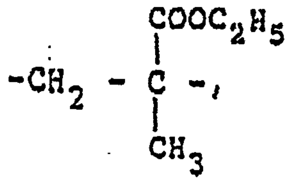

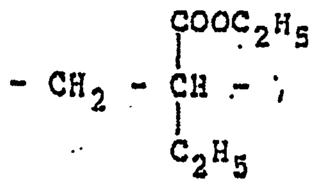

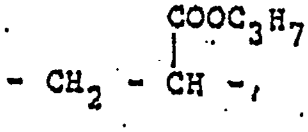

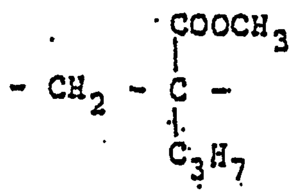

, or

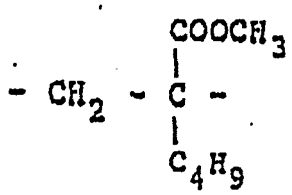

(ii) has an average molecular weight within the range of from about 100,000 to about 500,000, and (iii) is present in an amount within the range of from about 0.1 to about 4% by weight of the lipstick formulation, and (b) an oil dispersing agent for said acrylate polymer powder, and (B) from about 8 to about 35% by weight of a color intermediate which includes an oil dispersing agent.

**Patentansprüche**

1. Lippenstiftformulierung, die weich ist, aber gute Haftstruktur besitzt, cremig und leuchtend ist und gute Aufbringungs- und Farbabnutzungseigenschaften hat, mit etwa 40 bis etwa 80 Gew.-% wasserfreiem Basenzwischenprodukt, das ein oder mehrere Wachse und ein oder mehrere Öle umfaßt, etwa 8 bis 35 Gew.-% eines Farbzwischenproduktes, das ein Öldispergiermittel einschließt, und einem wasserfreien Acrylatpolymerpulver-Dispersionszwischenprodukt mit einem Acrylatpolymerpulver und einem Öldispergiermittel für das Acrylatpolymerpulver, worin das Acrylatpolymer (i) die sich wiederholende Einheit

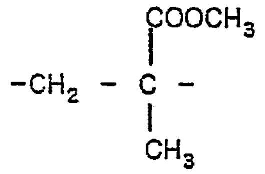

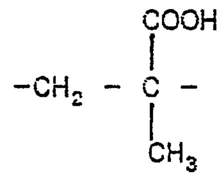

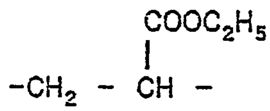

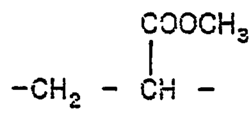

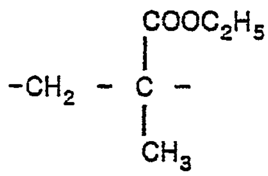

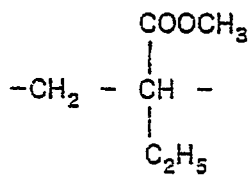

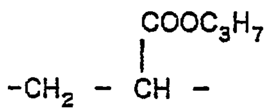

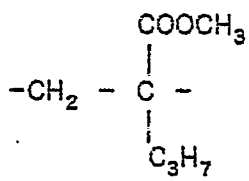

oder

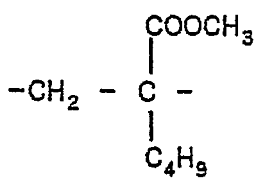

hat, (ii) ein mittleres Molekulargewicht im Bereich von etwa 100.000 bis etwa 500.000 hat und (iii) in einer Menge im Bereich von etwa 0,1 bis 4 Gew.-% der Lippenstiftformulierung vorliegt.

2. Lippenstiftformulierung nach Anspruch 1, worin das Acrylatpolymer in einer Menge im Bereich von etwa 0,3 bis etwa 2,5 Gew.-% der Lippenstiftformulierung vorliegt.

3. Lippenstiftformulierung nach Anspruch 1 oder 2, worin das Acrylatpolymer die sich wiederholende Einheit

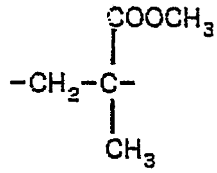

hat.

4. Lippenstiftformulierung nach einem der Ansprüche 1 bis 3, worin das wasserfreie Basenzwischenprodukt in einer Menge im Bereich von etwa 50 bis etwa 70 Gew.-% der Lippenstiftformulierung vorliegt.

5. Lippenstiftformulierung nach einem der Ansprüche 1 bis 4, worin das Basenzwischenprodukt ein oder mehrere größere Haftungsverstärker, ein oder mehrere Wachsanfühlverbesserer und Strukturfestiger, ein oder mehrere innere Haftungsverstärker, ein oder mehrere Anfühlverbesserer in Cremeform, ein oder mehrere Lippenbefeuchter, Weichmacher, Schmiermittel und/oder Gleitmittel, ein oder mehrere Kopplungsmittel für Wachse und Öle, gegebenenfalls ein oder mehrere Konservierungsmittel und gegebenenfalls ein oder mehrere Antioxidationsmittel einschließt.

6. Lippenstiftformulierung nach Anspruch 5, worin der äußere Haftungsverstärker in einer Menge im Bereich von etwa 5 bis etwa 20 Gew.-% der Gesamtformulierung vorliegt und Candelillawachs, Paraffinwachs, Carnaubawachs, Bienenwachs oder ein Gemisch hiervon ist.

7. Lippenstiftformulierung nach Anspruch 5 oder 6, worin der Wachsanfühlverbesserer und Strukturfestiger in einer Menge im Bereich von etwa 5 bis etwa 18 Gew.-% der Gesamtformulierung vorliegt und Cetylalkohol, Stearylalkohol, Lanolin, Lanolinalkohl, Glycerylmonostearat oder ein Gemisch hiervon ist.

8. Lippenstiftformulierung nach Anspruch 5, 6 oder 7, worin der innere Haftungsverstärker in einer Menge im Bereich von etwa 0,5 bis etwa 5 Gew.-% der Gesamtformulierung vorliegt und Paraffinwachs, Carnaubawachs oder Ozokeritwachs ist.

9. Lippenstiftformulierung nach Anspruch 5, 6, 7 oder 8, worin der Anfühlverbesserer in Cremeform in einer Menge im Bereich von etwa 1 bis etwa 10 Gew.-% der Gesamtformulierung vorliegt und hydriertes Pflanzenöl, Isopropyllanolat, Kakaobutter, acetylierter Lanolinalkohol, Lanolinderivat oder ein Gemisch hiervon ist.

10. Lippenstiftformulierung nach Anspruch 5, 6, 7, 8 oder 9, worin die Lippenbefeuchter, Weichmacher, Schmiermittel und/oder Gleitmittel in einer Menge im Bereich von etwa 3 bis 30%, bezogen auf die Gesamtformulierung vorliegen und Vaseline, Oleylalkohol, Rizinusöl, Mineralöl niedriger Viskosität, flüssiges Lanolin, Jojobaöl, Isopropylmyristat, Isopropylpalmitat, Squalen, Sesamöl oder ein Gemisch hiervon sind.

11. Lippenstiftformulierung nach Anspruch 5, 6, 7, 8, 9 oder 10, worin das Kopplungsmittel für Wachse und Öle in einer Menge im Bereich von etwa 6 bis etwa 15 Gew.-% der Gesamtformulierung vorliegt und Oleylalkohol, Dipropyldipelargonat, Rizinusöl oder ein Gemisch hiervon ist.

12. Lippenstiftformulierung nach einem der vorausgehenden Ansprüche, worin das Öldispergiermittel Rizinusöl, Mineralöl, Isopropylisostearat, Oleylalkohol, flüssiges Lanolin, Sesamöl, Isopropylmyristat, Isopropylpalmitat oder Squalen ist.

13. Verfahren zur Herstellung einer verbesserten Lippenstiftformulierung, die gute Aufbringungseigenschaften, Cremigkeit und Leuchtkraft sowie gute Farbabnutzung und Haftungsfestigkeit hat, indem man in eine wasserfreie Lippenstiftformulierung (A) ein wasserfreies Acrylatpolymerpulverdispersionszwischenprodukt, das (a) ein Acrylatpolymerpulver aufweist, worin das Acrylatpolymer (i) die sich wiederholende Einheit

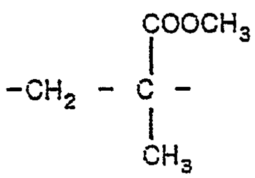

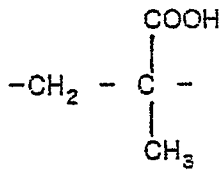

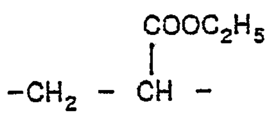

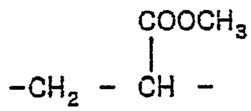

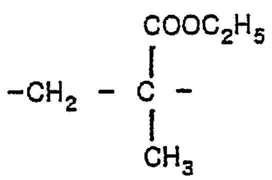

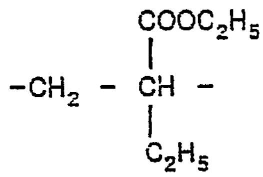

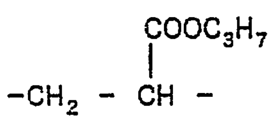

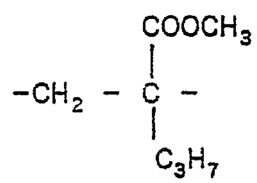

oder

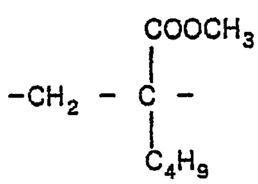

hat, (ii) ein mittleres Molekulargewicht im Bereich von etwa 100.000 bis etwa 500.000 hat und (iii) in einer Menge im Bereich von etwa 0,1 bis etwa 4 Gew.-% der Lippenstiftformulierung vorliegt, und (b) ein Öldispergiermittel für das Acrylatpolymerpulver enthält, und (B) etwa 8 bis etwa 35 Gew.-% eines Farbzwischenproduktes, das ein Öldispergiermittel einschließt, einarbeitet.

**Revendications**

1. Formulation de rouge à lèvres qui est molle, mais qui présente une bonne structure de bâton, est crémeuse et brillante, et qui possède de bonnes propriétés de pose et de tenue de la couleur, ladite formulation comprenant d'environ 40 à environ 80% en poids d'une phase de base anhydre comprenant une ou plusieurs cires et une ou plusieurs huiles; d'environ 8 à environ 35% en poids d'une phase colorante qui inclut un agent de dispersion huileux; et une phase de dispersion de poudre de polmère d'acrylate anhydre, compenant une poudre de polymère d'acrylate et un agent de dispersion huileux pour ladite poudre de polymère d'acrylate, le polymère d'acrylate (i) possédant le motif répétitif:

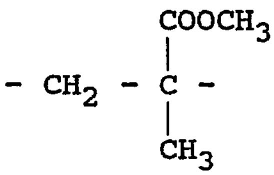 ,
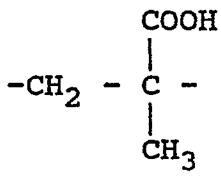 ,
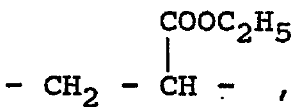 ,
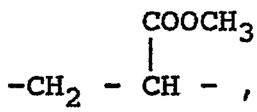 ,
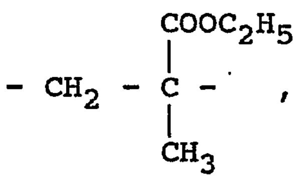 ,
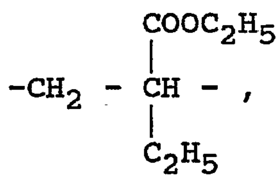 ,
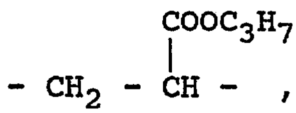 ,
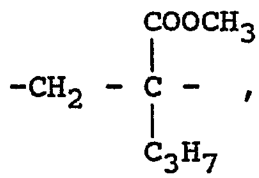 , ou
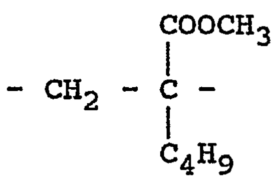 ,

(ii) ayant une masse moléculaire moyenne se situant dans la plage d'environ 100 000 à environ 500 000, et (iii) étant présent en une quantité se situant dans la plage d'environ 0,1 à environ 4% en poids de la formulation de rouge à lèvres.

2. Formulation de rouge à lèvres selon la revendication 1, dans laquelle la polymère d'acrylate est présent en une quantité se situant dans la plage d'environ 0,2 à environ 2,5% en poids de la formulation de rouge à lèvres.

3. Formulation de rouge à lèvres selon la revendication 1 ou 2, dans laquelle le polymère d'acrylate possède le motif répétitif:

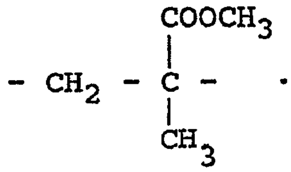

4. Formulation de rouge à lèvres selon l'une des revendications 1 à 3, dans laquelle la phase de base anhydre est présente en une quantité se situant dans la plage d'environ 50 à environ 70% en poids de la formulation de rouge à lèvres.

5. Formulation de rouge à lèvres selon l'une des revendications 1 à 4, dans laquelle la phase de base comprend un ou plusieurs agents renforçateurs externes de bâton, un ou plusieurs agents d'amélioration du toucher sous forme de cire et agents renforçateurs de structure, un ou plusieurs agents renforçateurs internes de bâton, un ou plusieurs agents d'amélioration du toucher sous forme de crème, un ou plusieurs agents d'humidification des lèvres, émollients, lubrifiants et/ou agents de coulage ou de glissement, un ou plusieurs agents de couplage pour les cires et les huiles, de façon facultative, un ou plusieurs agents de conservation et, de façon facultative, un ou plusieurs antioxydants.

6. Formulation de rouge à lèvres selon la revendication 5, dans laquelle l'agent renforçateur externe de bâton est présent en une quantité se situant dans la plage d'environ 5 à environ 20% en poids de la formulation totale, et est la cire de candelilla, la cire de paraffine, la cire de carnauba, la cire d'abeilles ou un mélange de celles-ci.

7. Formulation de rouge à lèvres selon la revendication 5 ou 6, dans laquelle l'agent d'amélioration du toucher sous forme de cire et l'agent renforçateur de structure sont présents en une quantité se situant dans la plage d'environ 5 à environ 18% en poids de la formulation totale, et sont l'alcool cétylique, l'alcool stéarylique, la lanoline, l'alcool lanolique, le monostéarate de glycéryle ou un mélange de ces substances.

8. Formulation de rouge à lèvres selon la revendication 5, 6 ou 7, dans laquelle l'agent renforçateur interne de bâton est présent en une quantité se situant dans la plage d'environ 0,5 à environ 5% en poids de la formulation totale, et est la cire de paraffine, la cire de carnauba ou la cire d'ozocérite.

9. Formulation de rouge à lèvres selon la revendication 5, 6, 7 ou 8, dans laquelle l'agent d'amélioration du toucher sous forme de crème est présent en une quantité se situant dans la plage d'environ 1 à environ 10% en poids de la formulation totale, et est une huile végétale hydrogénée, le lanolate d'isopropyle, le beurre de cacao, l'alcool lanolique acétylé, un dérivé de lanoline ou un mélange de ces substances.

10. Formulation de rouge à lèvres selon la revendication 5, 6, 7, 8 ou 9, dans laquelle les agents d'humidification des lèvres, les émollients, les lubrifiants et/ou les agents de coulage ou de glissement sont présents en une quantité se situnat dans la plage d'environ 3 à environ 30% sur la base de la formulation totale, et sont le blanc de pétrolatum, l'alcool oléylique, l'huile de ricin, une huile minérale de faible viscosité, la lanoline liquide, l'huile de jojoba, le myristate d'isopropyle, le palmitate d'isopropyle, le squalane, l'huile de sésame ou un mélange de ces substances.

11. Formulation de rouge à lèvres telle que définie à la revendication 5, 6, 7, 8, 9 ou 10, dans laquelle l'agent de couplage pour les cires et les huiles est présent en une quantité se situant dans la plage d'environ 6 à environ 15% en poids de la formulation totale, et est l'alcool oléylique, le dipélargongate de dipropyle, l'huile de ricin ou un mélange de ces substances.

12. Formulation de rouge à lèvres selon l'une des revendications précédentes, dans laquelle ledit agent de dispersion huileux est l'huile de ricin, une huile minérale, l'isostéarate d'isopropyle, l'alcool oléylique, la lanoline liquide, l'huile de sésame, le myristate d'isopropyle, le palmitate d'isopropyle ou le squalène.

13. Procédé de préparation d'une formulation de rouge à lèvres améliorée qui présente de bonnes propriétés de pose, du velouté et de la brillance, et de bonnes propriétés de tenue de la couleur et de résistance de bâton, ledit procédé consistant à incorporer dans une formulation de rouge lèvres anhydre:

(A) une phase de dispersion d'une poudre de polymère d'acrylate anhydre comprenant:

(a) une poudre de polymère d'acrylate dans laquelle le polymère d'acrylate (i) possède le motif répétitif:

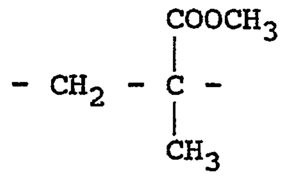 , 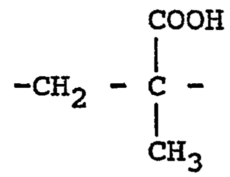 ,

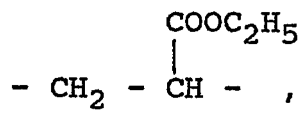 , 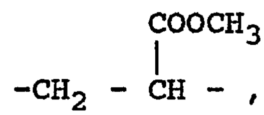 ,

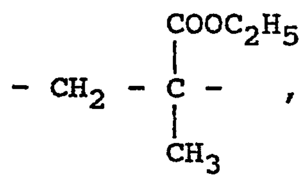 , 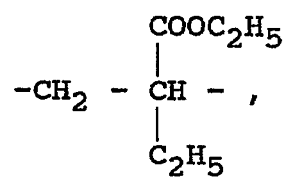 ,

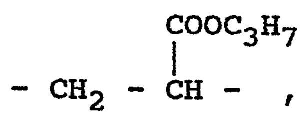 , 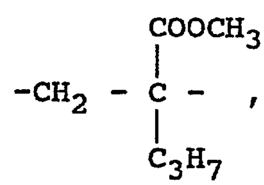 , ou

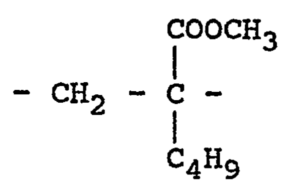 ,

(ii) possède une masse moléculaire moyenne se situant dans la plage d'environ 100 000 à environ 500 000, et (iii) est présent en une quantité se situant dans la plage d'environ 0,1 à environ 4% en poids de la formulation de rouge à lèvres, et

(b) un agent de dispersion huileux pour ladite poudre de polymère d'acrylate, et

(B) d'environ 8 à environ 35% en poids d'une phase colorante qui inclut un agent de dispersion huileux.